# EUROPEAN PATENT SPECIFICATION

(11) **EP 0 461 580 B1**
(45) Date of publication and mention of the grant of the patent: **10.01.1996**
(21) Application number: 91109470.4
(22) Date of filing: 10.06.1991
(51) Int. Cl.: C07C 69/14, C07C 67/04

(54) **Process for producing cyclohexyl acetate**
Verfahren zum Herstellen von Cyclohexylacetat
Procédé de production de l'acétate de cyclohexyle

(30) Priority: 11.06.1990 JP 149965/90; 15.06.1990 JP 155253/90
(43) Date of publication of application: 18.12.1991
(73) Proprietor: MITSUI TOATSU CHEMICALS, Inc., Chiyoda-Ku Tokyo 100 (JP)
(72) Inventor: Inoue, Kaoru, Totsuka-ku, Yokohama-shi, Kanagawa-ken (JP); Iwasaki, Masao, Yokohama-shi, Kanagawa-ken (JP); Matsui, Kazuaki, Kamakura-shi, Kanagawa-ken (JP)
(74) Representative: VOSSIUS & PARTNER

(56) References cited:
- FR-A- 2 022 349

## Description

The present invention relates to a novel process for producing cyclohexyl acetate.

Heretofore, as processes for producing cyclohexyl acetate, an esterification method of acetic acid with cyclohexanol and a reaction of cyclohexanol with acetic anhydride have been generally known.

However, both processes use cyclohexanol as a starting material, and cyclohexanol presents a problem as to the method for preparation thereof and has various other drawbacks. In addition, the esterification reaction is an equilibrium reaction so that it is difficult to produce the ester in high yield and moreover means are necessary for removing the water formed as a by-product.

In the method of using acetic anhydride, although the yield of the ester is high, there are many various drawbacks, for example: acetic anhydride is expensive and in addition, one molecule of acetic acid is released and it is difficult to convert the acetic acid into the anhydride.

For the purposes of solving these problems, some methods for producing cyclohexyl acetate by an addition reaction of cyclohexene with acetic acid have been recently proposed. For example, JP-A-254634/1989 discloses a method for producing cyclohexyl acetate by the reaction of cyclohexene with acetic acid in the presence of a strongly acidic cation exchange resin as a catalyst.

According to this method, the reaction should be effected at 130°C for 5 hours. However, in general, the heat resistant temperature of such strongly acidic cation exchange resins are inherently about 100°C, and even highly heat resistant resins cannot withstand a temperature higher than 160°C. Thus, when such cation ion exchange resins are used, a high reaction temperature and a long reaction time are required.
In addition, these cation ion exchange resins have also drawbacks that the mechanical strength is low and the resins are easily broken so that the stability as a catalyst is unreliable to a great extent. Furthermore, the catalytic activity of these resins is low.

JP-A-313447/1989 discloses an addition reaction of acetic acid with cyclohexene in the presence of both ZSM-5, a high silica content-zeolite, as a catalyst and water. According to this method, the reaction temperature is 120°C and the reaction time is 4 hours resulting in the formation of cyclohexanol in a 12.5 % yield and cyclohexyl acetate in a yield of at most 65 %. The reaction temperature is high and the catalytic activity is low.

FR-A-2 022 349 discloses a process for the preparation of esters of carboxylic acids by reaction of acetic acid with cyclohexene in the presence of 10-molybdophosphoric acid.

The technical problem underlying the present invention is to provide an efficient process for the production of cyclohexyl acetate from cyclohexene and acetic acid in a high conversion and high selectivity within a short time and to provide a catalyst useful for this process.

Another problem of the present invention is to provide a catalyst serving for producing cyclohexyl acetate effectively at a low temperature region.

A further problem of the present invention is to provide a catalyst capable of producing cyclohexyl acetate stably even at a high temperature region. According to the present invention, these problems are solved by a process for producing cyclohexyl acetate from cyclohexene and acetic acid which comprises reacting acetic acid with cyclohexene in the presence of at least one heteropolyacid mainly composed of oxides of tungsten which has water of crystallization adjusted to an amount on average of 3.0 molecules or less per one molecule of the heteropolyacid.

According to the present invention, the heteropolyacids mainly composed of oxides of tungsten used as catalysts are preferably heteropolyacids of the general formula

(M₁)ₐ(M₂)_{b}(W)_{c}(O)_{d}(H)ₑ

where M₁ and M₂ are independently metal elements, W is tungsten, O is oxygen, H is hydrogen, a is an integer of 1 or 2, b is an integer of 0, 1 or 2, c is a positive integer of 20 or less, d is a positive integer of 100 or less, and e is a positive integer of 10 or less.

In the above-mentioned general formula, M₁ is preferably an element selected from the group consisting of P, Si, Co, Mn, Ni, As, Ti, Fe, V and B, and M₂ is V. More preferably, the heteropolyacid is tungstophosphoric acid and/or tungstosilicic acid. The most easily available heteropolyacids are, for example, dodecatungstosilicic acid (SiW₁₂O₄₀H₄), heteropolyacids produced by replacing one or more of tungsten atoms of dodecatungstosilicic acid with vanadium atoms, dodecatungstophosphoric acid (PW₁₂O₄₀H₄), and heteropolyacids produced by replacing one or more of tungsten atoms with vanadium atoms, and the like. However, the process of the present invention is not limited to these heteropolyacids.

In the process of the present invention the heteropolyacids mainly composed of oxides of tungsten may be those containing no water of crystallization and/or those containing water of crystallization. The heteropolyacids mainly composed of oxides of tungsten have water of crystallization adjusted to the amount of average 3.0 molecules or less per one molecule of the heteropolyacid.

In addition, it is much more preferable in the process of the present invention to use a heteropolyacid mainly composed of oxides of tungsten the average amount of water of crystallization of which is 3.0 molecules or less per one molecule of the heteropolyacid resulting from adjusting the amount of said water by heating a heteropolyacid mainly composed of tungsten oxide at a temperature range of from 150°C to 500°C, or by heating a heteropolyacid mainly composed of tungsten oxide in a gas stream inert to the heteropolyacid.

When the process of the present invention is carried out in the presence of the heteropolyacid,the amount of water of crystallization of which has been adjusted to 3.0 molecules or less per one molecule of the heteropolyacid, it is preferable that the amount of water other than water of crystallization of the heteropolyacid in the reaction system is 30.0 molecules or less per one molecule of the heteropolyacid.

In the process of the present invention, it is not particularly necessary to purify the starting materials, that is, acetic acid and cyclohexene, and acetic acid and cyclohexene of commercial grade may be used. It is preferable to remove water from these starting materials.

Heteropolyacids mainly composed of oxides of tungsten usually contain water of crystallization. In the present invention, "water of crystallization" of the heteropolyacids is a general term for water contained in heteropolyacids when they are prepared or commercially sold.

Concretely it is, for example, water attached to or taken up into the heteropolyacid crystals when heteropolyacids are prepared in a water-containing solvent and then precipitated by using a non-solvent or the like, or water taken up into the heteropolyacid by adsorption, deliquescence or the like.

For example, a commercially available dodecatungstosilicic acid can be represented by the general formula, H₄SiW₁₂O₄₀·nH₂O and a commercially available dodecatungstophosphoric acid can be represented by the general formula, H₃PW₁₂O₄₀·nH₂O. The n·H₂O comprises water of crystallization, and water molecules taken up into or adsorbed by or contained in the crystal structure.

Therefore, in the process of the present invention the heteropolyacids represented by the above-mentioned general formulae indicate that they have n molecules of water of crystallization. The n is an average value so that it is not always an integer, but a real number. The n of commercially available heteropolyacids usually ranges from about 25 to about 30. The n can be higher than these values due to the adsorption of water or the like.

The content of water of crystallization contained in those heteropolyacids can be easily reduced i.e. the value of n can be decreased, by a dehydration treatment such as heating and the like. It is also possible to reduce the value of n to zero. In addition, it is also possible to increase easily again the value of n by causing the dehydrated heteropolyacid to adsorb water.

According to the following methods, water of crystallization contained in heteropolyacids mainly composed of oxides of tungsten used as a catalyst for the process of the present invention may be reduced to 3 molecules or less per one molecule of the heteropolyacid.

Easily available heteropolyacids mainly composed of oxides of tungsten usually contain several tens of moles of water of crystallization. A method for reducing the water of crystallization to an average of 3 molecules or less per one molecule of the heteropolyacid is not particularly limited in the present invention, but a dehydration treatment by heating etc. may be mentioned as an easily operable method.

As the dehydration treatment by heating, for example, heating in an ordinary electric furnace (muffle furnace) is recommendable. Naturally, according to the present invention, the method is not limited to the heating by this apparatus.

Neither is the heating temperature particularly limited and any temperature may be used as long as water of crystallization can be reduced to an average of 3.0 molecules or less per one molecule of the heteropolyacid. For the effective operation, it is preferable to effect the dehydration at a temperature of 80°C - 500°C, more preferably, 200°C - 450°C. At a lower temperature, the dehydration is difficult while heteropolyacid anhydrides are formed at 550°C or higher.

The heating time is not particularly limited as long as the amount of water of crystallization lies in the above-mentioned range. For example, when an ordinary electric furnace is used and the dehydration is conducted at about 300°C, the above-mentioned range of amount of water of crystallization can be attained in about 3 hours. When a heteropolyacid is used as a catalyst, which is prepared by heating and dehydrating the heteropolyacid in an inert gas stream at the heat-dehydrating temperature and controlling the amount of water of crystallization to an average of 3.0 molecules or less per one molecule of the heteropolyacid, the activity of the resulting catalyst is much higher. This operation provides a very effective catalyst.

The gas used during the heat-dehydration operation may be any gas as long as it is inert to the heteropolyacid mainly composed of oxides of tungsten at the heating temperature.

In addition, even if the gas is liquid or solid at ordinary temperatures, it may be used, provided that it is in a gaseous state at the heating temperature and inert to the heteropolyacid.

Examples of suitable gases include air, argon, nitrogen, helium, hydrogen, aliphatic hydrocarbons, aromatic hydrocarbons and the like, but naturally are not limited thereto. In addition, the flow rate of inert gas is not critical.

The space velocity in the apparatus in terms of gas volume at the heating temperature is preferably 0.2 - 20 vol/hr·vol.

According to the process of the present invention, even when the heteropolyacid contains 20-30 molecules of water of crystallization per one molecule of the heteropolyacid which are ordinary amounts of water of crystallization of the heteropolyacid, it exhibits a good catalytic activity.

However, when the amount of water is controlled to an average of 3.0 molecules or less per one molecule of the heteropolyacid by dehydration or the like, the resulting heteropolyacid exhibits a particularly high catalytic activity. In addition, since this catalyst is thermally very stable, the process of the present invention can be sufficiently stably carried out at an elevated temperature.

According to the process of the present invention, the reaction of acetic acid with cyclohexene to form cyclohexyl acetate proceeds as an addition reaction.

The reaction may be carried out in a liquid phase homogeneous system, or in a solid phase-gas phase or a solid phase-liquid phase heterogeneous system or the like.

The process of the present invention may be effected under any pressure condition such as normal pressure, reduced pressure, and elevated pressure. When the reaction is effected at a high temperature, a liquid phase reaction may be conducted at an elevated pressure.

The operation type of the reaction is not particularly limited. A continuous process, batch process or semi-batch process may be employed.

Inert solvents or diluents may be added to the catalyst and reaction agents (the starting materials and product ).

Examples of suitable inert solvents and diluents include saturated aliphatic hydrocarbons such as n-butane, n-pentane, n-hexane, n-heptane, n-octane, n-nonane, n-decane, isomers thereof and the like; aromatic hydrocarbons such as benzene, toluene, ethylbenzene, xylene, anisole, cumene, nitrobenzene and the like; saturated alicyclic hydrocarbons such as cyclopentane, alkyl-substituted cyclopentanes, alkoxy-substituted cyclopentanes, nitro-substituted cyclopentanes, cyclohexane, alkyl-substituted cyclohexanes, alkoxysubstituted cyclohexanes, nitro-substituted cyclohexanes, cycloheptane, alkyl-substituted cycloheptanes, alkoxysubstituted cycloheptanes, nitro-substituted cycloheptanes, cyclooctane, alkyl-substituted cyclooctanes, alkoxy-substituted cyclooctanes, nitro-substituted cyclooctanes and the like; nitrogen, air,argon, helium and the like.

When the reaction is carried out at an elevated pressure, it is possible to pressurize inert nitrogen, argon or the like into the reaction agents and catalyst in advance and carry out the reaction in a liquid phase reaction system.

In the process of the present invention, the ratio of acetic acid to cyclohexene charged for effecting the reaction is not particularly limited. The molar ratio of acetic acid to cyclohexene is usually in the range of 0.1 to 100. For example, in order to attain a high conversion of acetic acid, it is desirable to effect the reaction at a molar ratio of acetic acid to cyclohexene less than 1 while in order to attain a high conversion of cyclohexene, it is desirable to effect the reaction at a molar ratio of acetic acid to cyclohexene more than 1.

For carrying out the process of the present invention, the amount of the catalyst to be added is not particularly critical. However, it is advisable in the case of a batch process to use the catalyst in an amount of 0.1 - 100 % by weight, preferably 1 - 50 % by weight based on the weight of cyclohexene fed.

When the amount of the catalyst is less than the above-mentioned amount, the reaction proceeds slowly while at an amount exceeding the above-mentioned amount the reaction proceeds sufficiently but is not preferable from an economical point of view. However, the process of the present invention naturally can be effected outside of the above-mentioned range.

Upon carrying out the process of the present invention, the manner in which the catalyst and reaction agents are fed is not particularly limited. The catalyst, acetic acid and cyclohexene may be simultaneously fed. The catalyst may be dissolved or suspended in an other solvent and then fed to a reaction vessel. In addition, there are various methods, for example, before the reaction, acetic acid and the catalyst are mixed in advance and then cyclohexene is added, or the catalyst and cyclohexene are mixed and then acetic acid is added.

When effecting the process of the present invention with a heteropolyacid catalyst mainly composed of oxides of tungsten containing an average of 3.0 molecules of water of crystallization or less per one molecule of the heteropolyacid, the amount of water in the reaction system particularly affects the reaction.

In particular, in order to obtain a catalytic activity higher than that of a usually commercially available heteropolyacid containing several tens of molecules of water of crystallization per one molecule of the heteropolyacid, it is necessary to control the amount of water other than water of crystallization contained in the heteropolyacid to 30 molecules or less per one molecule of the heteropolyacid used.

When the amount of water is higher than that, dehydration of water of crystallization in the heteropolyacid fails to give an effective result.

In such a case, the water content in the acetic acid and cyclohexene is at most 100 ppm so that, when the amount of the catalyst added to the reaction system is a few percents based on the reaction system, the water content (excluding water of crystallization) in the reaction system never exceeds the above-mentioned value if commercially available acetic acid and cyclohexene are used in the process of the present invention.

The reaction temperature in the process of the present invention is not particularly limited. The reaction may be effected within a wide range of temperatures. The preferred range is 0°C - 400°C, more preferably 30°C - 200°C. If the reaction temperature is too low, the reaction velocity is lowered. On the other hand, if the reaction temperature is too high, reaction agents such as cyclohexene are liable to be thermally deteriorated. Thus, high temperatures are not economical.

The reaction time varies depending upon the amount of catalyst and/or the reaction temperature. When the amount of catalyst is high and the temperature is high, the reaction time is very short, for example, about 0.1 sec. or less. On the other hand, when the amount of catalyst is low and the temperature is low, the reaction time is long, for example, about 24 hours.

After completion of the reaction, the final product can be recovered by ordinary separation procedures such as distillation.

Examples of various methods for carrying out the process of the present invention are as shown below. The methods are not critical, but the following are some examples of easily operable methods though the present invention is not limited thereto.
(1) A glass flask equipped with a stirrer is charged with prescribed amounts of the catalyst, acetic acid and cyclohexene, if desired, together with a diluent such as a solvent and the like and if necessary, a reflux condenser is fitted to the flask. Then the reaction is effected by heating with stirring.
(2) An autoclave is charged with prescribed amounts of the catalyst, acetic acid and cyclohexene, if desired, together with a medium such as nitrogen, argon and the like. Then the reaction is conducted with stirring and heating.
(3) Into a reactor previously kept at a prescribed temperature and pressure are continuously introduced prescribed amounts of the catalyst, cyclohexene and acetic acid, if desired, together with a diluent such as a solvent and the like to carry out the reaction.

According to the process of the present invention, cyclohexyl acetate can be prepared very efficiently by an addition reaction of acetic acid with cyclohexene in the presence of a heteropolyacid mainly composed of oxides of tungsten, and further, drawbacks of conventional catalysts such as low catalytic activity, low heat resistance and the like can be avoided. The catalyst used in the process of the present invention is effective even at low temperatures and stable even at high temperatures.

In particular, when the amount of water of crystallization of the heteropolyacid is controlled to an average of 3 molecules or less per one molecule of the heteropolyacid, the reaction proceeds with a very high efficiency. Even under very mild conditions such as low temperature and within a short time, for example, about one hour, cyclohexyl acetate can be produced in high selectivity and in high conversion.

Moreover, even under high temperature conditions, the process can be stably and effectively carried out.

Cyclohexyl acetate may be used as a starting material for cyclohexanol which is useful as an intermediate for the production of phenol, nylon and adipic acid. It may be used as a cycloalkanol carboxylic acid ester useful as a special solvent.

The present invention is explained in the following in more detail referring to examples.

Legend in the following tables:
- Cy - Hex: Cyclohexene
- Cy - HexOAc: Cyclohexyl acetate
- Ar: Argon
- He: Helium

(1) Quantitative Measurement of the Reaction Product:
   After conducting the reaction for a prescribed time at a prescribed temperature, the reaction liquid was cooled to room temperature and subjected to gas chromatography to measure the reaction product quantitatively.
(2) Quantitative Measurement of Water of Crystallization contained in the catalyst:
   The amount of water of crystallization contained in the heteropolyacid mainly composed of oxides of tungsten was determined based on an anhydrous heteropolyacid (the amount of water of crystallization being zero) produced by heating and dehydrating the heteropolyacid at 500°C until the weight became constant and did not decrease any more.

### Comparative Example 1

A 70 ml three-necked flask equipped with a magnetic stirrer and a reflux condenser was charged with commercial dodecatungstophosphoric acid (H₄PW₁₂O₄₀ ·28H₂O) 2.0 g, commercial 99.5 % acetic acid (manufactured by Kokusan Kagaku K.K., special grade) 20.5 g and commercial 98 % cyclohexene (manufactured by Tokyo Kasei K.K., special grade) 4.5 g. The flask was placed in an oil bath and heated at 70°C for 1.5 hours with stirring to effect the reaction.

After the reaction, stirring was stopped and the flask was taken out of the oil bath and cooled to room temperature. Then the reaction liquid was quantitatively analyzed.

The result is as shown in Table 1, that is, yield of cyclohexyl acetate was 33.4 % (selectivity being 97.3 %) and conversion of cyclohexene was 34.3 %.

### Comparative Example 2

The procedure of Comparative Example 1 was repeated to produce cyclohexyl acetate from cyclohexene and acetic acid under the same conditions except that the reaction tame was 3 hours. As a result, the amount of the product, cyclohexyl acetate, increased as the reaction time increased as shown in Table 1.

### Comparative Example 3

The procedure of Comparative Example 1 was repeated to carry out the reaction of acetic acid and cyclohexene under the same conditions including catalyst amount except that the catalyst was replaced with a commercially available dodecatungstosilicic acid (H₄SiW₁₂O₄₀·25H₂O). The result is as shown in Table 1.

### Comparative Example 4

The procedure of Comparative Example 1 was repeated under the same reaction conditions except that 2.0 g of H-ZSM5 zeolite was used as a catalyst in place of dodecatungstophosphoric acid. Cyclohexyl acetate was not formed and the starting materials, cyclohexene and acetic acid, were recovered.

**Table 1**

| | Cy - Hex | Cy - HexOAc | |
|---|---|---|---|
| | Conversion (%) | Yield (%) | Selectivity (%) |
| Comparative Example 1 | 34.3 | 33.4 | 97.3 |
| Comparative Example 2 | 50.6 | 50.2 | 99.2 |
| Comparative Example 3 | 57.2 | 54.8 | 95.8 |

### Example 1

### Preparation of dehydrated dodecatungstophosphoric acid catalysts by heating:

As shown in Table 2 (infra), the above-mentioned commercially available dodecatungstophosphoric acid was heated and dehydrated in an electric furnace (muffle furnace) at a prescribed temperature for a prescribed time. As a result, the compositions of water of crystallization in dodecatungstophosphoric acid are as shown in Table 2 (infra). The number of molecule of water of crystallization per one molecule of the dodecatungstophosphoric acid thus dehydrated is designated as n' in the table.

**Table 2**

| | Heating temperature (°C) | Heating time (hr.) | n' |
|---|---|---|---|
| Catalyst 1 | 350 | 3.0 | 0 |
| Catalyst 2 | 400 | 3.0 | 0 |
| Catalyst 3 | 300 | 3.0 | 0 |
| Catalyst 4 | 250 | 1.0 | 1.4 |
| Catalyst 5 | 250 | 0.5 | 3.0 |
| Catalyst 6 | 200 | 0.25 | 4.8 |
| Catalyst 7 | 120 | 1.0 | 8.0 |

### Example 2

The reaction of acetic acid with cyclohexene was effected by using the same apparatus as that of Comparative Example 1 and under the same conditions as those of Comparative Example 1 except that the above-mentioned catalyst 1 was used as a heteropolyacid catalyst. As shown in Table 3, the catalytic activity was markedly improved.

### Example 3

The reaction of acetic acid with cyclohexene was effected using the same apparatus and conditions as those of Example 2 except that the above-mentioned catalyst 2 was used as a heteropolyacid catalyst. As shown in Table 3, when the dehydration temperature for preparing the catalyst was elevated, the catalytic activity was hardly affected. It was confirmed that the catalytic activity was very high.

### Example 4

The reaction of Example 2 was repeated except that catalyst 3 was used. As shown in Table 3, the reaction result was similar to those for catalyst 1 and catalyst 2. The facts in Examples 2 - 4 show that the amount of water of crystallization of a heteropolyacid is more important than the dehydration temperature for remarkably improving the activity of the catalyst by dehydrating the heteropolyacid mainly composed of oxides of tungsten.

### Example 5

The procedure of Example 2 was repeated except that catalyst 4 was used as a catalyst. The result is shown in Table 3.

### Example 6

The procedure of Example 2 was repeated except that catalyst 5 was used as a catalyst. The result is shown in Table 3.

### Comparative Example 5

The reaction of acetic acid with cyclohexene was effected under the same conditions as in Example 2 except that catalyst 6 was used as a catalyst. As can be seen from the result shown in Table 3, an improvement in catalytic activity due to dehydration of the heteropolyacid was not observed.

### Comparative Example 6

The reaction was effected under the same conditions as in Example 2 except that catalyst 7 was used as a catalyst. As can be seen from the result shown in Table 3, the catalyst exhibited a catalytic activity similar to that of a commercially available dodecatungstophosphoric acid.

### Example 7

The reaction of acetic acid with cyclohexene was conducted under the same conditions as in Example 2 except that only 0.5 g of catalyst 1 was used as compared with 2 g in Example 2. The result is shown in Table 3.

### Example 8

The reaction was effected under the same conditions as in Example 7 except that the reaction time was 3 hours. As can be seen from the result shown in Table 3, as the reaction time increased, the amount of produced cyclohexyl acetate increased and no deterioration of the catalyst occurred.

### Example 9

A 200 ml autoclave was charged with 2.0 g of catalyst 1, 61.5 g of acetic acid and 13.5 g of cyclohexene. Nitrogen gas was fed to the autoclave until the pressure reached 5 kg/cm² (gauge), and then, the cyclohexyl acetate producing reaction was effected at 100°C for 1 hour. The result is shown in Table 3.

### Example 10

The procedure of Example 9 was repeated to effect the reaction of acetic acid with cyclohexene except that the reaction temperature was 130°C and the reaction time was 0.5 hour. The result is shown in Table 3, that is, cyclohexyl acetate was produced in high yield within a short time.

**Table 3**

| | Catalyst | n' | Cy - Hex | Cy - HexOAc | |
|---|---|---|---|---|---|
| | | | Conversion (%) | Yield (%) | Selectivity (%) |
| Example 2 | 1 | 0 | 81.7 | 80.6 | 97.3 |
| Example 3 | 2 | 0 | 80.9 | 80.5 | 99.5 |
| Example 4 | 3 | 0 | 81.2 | 80.5 | 99.1 |
| Example 5 | 4 | 1.4 | 82.6 | 80.8 | 97.8 |
| Example 6 | 5 | 3.0 | 70.5 | 68.0 | 96.5 |
| Comparative Example 5 | 6 | 4.8 | 9.4 | 8.9 | 94.9 |
| Comparative Example 6 | 7 | 8.0 | 37.1 | 35.1 | 94.5 |
| Example 7 | 1 | 0 | 41.3 | 40.2 | 97.4 |
| Example 8 | 1 | 0 | 63.4 | 62.3 | 98.2 |
| Example 9 | 1 | 0 | 89.5 | 88.9 | 99.3 |
| Example 10 | 1 | 0 | 91.2 | 90.4 | 99.1 |

The amount of catalyst in each of the Examples other than Examples 7 and 8 was 2.0 g, and in Examples 7 and 8 0.5 g. n' denotes the number of molecules of water of crystallization contained in one molecule of the dehydrated heteropolyacid catalyst.

### Example 11

### Preparation of dehydrated heteropolyacid catalyst:

The above-mentioned commercially available dodecatungstosilicic acid (containing 25 molecules of water of crystallization per one molecule of the heteropolyacid) was placed in a muffle furnace and subjected to heating and dehydration at the prescribed temperature for the prescribed time as shown in Table 4. The number of molecules of water of crystallization contained in the dodecatungstosilicic acid thus dehydrated per one molecule of said heteropolyacid is designated as n', which is shown in Table 4.

**Table 4**

| | Heating temperature (°C) | Heating time (hr.) | n' |
|---|---|---|---|
| Catalyst 8 | 370 | 3.0 | 0 |
| Catalyst 9 | 420 | 1.0 | 0 |
| Catalyst 10 | 300 | 3.0 | 0 |
| Catalyst 11 | 250 | 2.0 | 1.7 |
| Catalyst 12 | 250 | 0.75 | 3.0 |
| Catalyst 13 | 250 | 0.5 | 4.0 |
| Catalyst 14 | 180 | 0.25 | 5.8 |
| Catalyst 15 | 150 | 0.75 | 12.7 |
| Catalyst 16 | 100 | 1.0 | 21.5 |

### Example 12

The reaction of acetic acid with cyclohexene was effected by repeating the procedure of Comparative Example 3 except that catalyst 8 (2.0 g fed) was used in place of the commercially available dodecatungstosilicic acid. As can be seen from the result shown in Table 5, a very high catalytic activity was achieved.

### Example 13

The procedure of Example 12 was repeated except that catalyst 9 was used in place of said catalyst. As can be seen from the result shown in Table 5, the reaction result was hardly affected by elevating the dehydration temperature.

### Example 14

The procedure of Example 12 was repeated except that catalyst 10 was used in place of said catalyst. As can be seen from the result shown in Table 5, the reaction result was hardly affected by lowering the dehydration temperature when preparing the catalyst.

### Example 15

The reaction of acetic acid with cyclohexene was effected under the same conditions as in Example 12 except that catalyst 11 was used. The result is shown in Table 5.

### Example 16

The procedure of Example 12 was repeated except that catalyst 12 was used. The result is shown in Table 5.

### Comparative Example 7

The cyclohexyl acetate production reaction was effected by repeating the procedure of Example 12 except that catalyst 13 was used. The result is shown in Table 5.

### Comparative Example 8

The procedure of Example 12 was repeated except that catalyst 14 was used. As can be seen from the result shown in Table 5, the reaction result was lowered in a similar manner to Comparative Example 7.

### Comparative Example 9

The procedure of Example 12 was repeated except that catalyst 15 was used. As can be seen from the result shown in Table 5, no catalyst activation effect due to the dehydration of the heteropolyacid was present.

### Comparative Example 10

The procedure of Example 12 was repeated except that catalyst 16 was used. As shown in Table 5, no effect due to the dehydration of the heteropolyacid was present.

In view of the results of Examples 12 - 16 and Comparative Examples 7 - 10, it is noted that when the average amount of water of crystallization contained in a heteropolyacid is 3.0 molecules or less per one molecule of the heteropolyacid, the heteropolyacid exhibits a very high catalytic activity in the cyclohexyl acetate forming reaction of acetic acid with cyclohexene.

### Example 17

The procedure of Example 12 was effected except that 0.5 g of catalyst 8 was used. The result is shown in Table 5.

### Example 18

The procedure of Example 17 was repeated except that the reaction time was 3 hours. As can be seen from the result shown in Table 5, as the reaction time increases, the amount of cyclohexyl acetate produced increases. This fact indicates that the catalyst is not deteriorated.

### Example 19

A 200 ml autoclave was charged with 2.0 g of catalyst 8, 61.5 g of acetic acid, 13.5 g of cyclohexene. Nitrogen gas was fed into the autoclave until the pressure reached 5 kg/cm² (gauge). Then the cyclohexyl acetate production reaction was carried out at 100°C for one hour.
The result is shown in Table 5.

### Example 20

The procedure of Comparative Example 7 was repeated except that the reaction temperature was 130°C and the reaction time was 0.5 hour. The result is shown in Table 5. It indicates that cyclohexyl acetate was produced in high yield within a short time.

**Table 5**

| | Catalyst | n' | Cy - Hex | Cy - HexOAc | |
|---|---|---|---|---|---|
| | | | Conversion (%) | Yield (%) | Selectivity (%) |
| Example 12 | 8 | 0 | 87.2 | 86.2 | 98.9 |
| Example 13 | 9 | 0 | 86.8 | 86.0 | 99.1 |
| Example 14 | 10 | 0 | 85.9 | 85.3 | 99.3 |
| Example 15 | 11 | 1.7 | 87.5 | 85.7 | 97.9 |
| Example 16 | 12 | 3.0 | 72.8 | 71.6 | 98.3 |
| Comparative Example 7 | 13 | 4.0 | 14.2 | 9.1 | 64.8 |
| Comparative Example 8 | 14 | 5.8 | 15.6 | 12.1 | 77.6 |
| Comparative Example 9 | 15 | 12.7 | 56.2 | 51.8 | 92.8 |
| Comparative Example 10 | 16 | 21.5 | 55.8 | 51.0 | 91.4 |
| Example 17 | 8 | 0 | 43.7 | 42.5 | 97.3 |
| Example 18 | 8 | 0 | 73.8 | 72.9 | 98.9 |
| Example 19 | 8 | 0 | 91.6 | 91.1 | 99.5 |
| Example 20 | 8 | 0 | 95.2 | 94.4 | 99.2 |

The amount of catalyst used in each of Examples other than Examples 17 and 18 was 2.0 g while that in each of Examples 17 and 18 was 0.5 g.

### Example 21

### Preparation of dehydrated dodecatungstophosphoric acid Catalyst (2):

The above-mentioned commercially available dodecatungstophosphoric acid was placed in a muffle furnace having an inner volume of 8 liter, which a gas or the like can be introduced into and discharged from, and subjected to heating and dehydration at a prescribed temperature for a prescribed time by introducing various gases into the muffle at prescribed flow rates as shown in Table 6. The number of molecule of water of crystallization contained in the tungstophosphoric acid thus treated per one molecule of the tungstophosphoric acid is designated as n' in Table 6.

**Table 6**

| | Gas | Flow rate (Nl/hr.) | Temperature (°C) | Time (hr.) | n' |
|---|---|---|---|---|---|
| Catalyst 17 | Ar | 5.0 | 350 | 3 | 0 |
| Catalyst 18 | Ar | 5.0 | 400 | 1 | 0 |
| Catalyst 19 | Ar | 5.0 | 300 | 3 | 0 |
| Catalyst 20 | Ar | 5.0 | 250 | 1 | 1.3 |
| Catalyst 21 | Ar | 5.0 | 250 | 0.5 | 2.9 |
| Catalyst 22 | Ar | 35.0 | 350 | 3 | 0 |
| Catalyst 23 | Air | 35.0 | 350 | 3 | 0 |
| Catalyst 24 | He | 35.0 | 350 | 3 | 0 |

### Example 22

The reaction of acetic acid with cyclohexene under the same conditions as in Comparative Example 1 was carried out except that catalyst 17 (added amount, 2.0g) was used in place of the above-mentioned commercially available dodecatungstophosphoric acid. The result is shown in Table 7. It indicates that the dodecatungstophosphoric acid was converted to a very highly active catalyst by heating and dehydrating dodecatungstophosphoric acid in an argon gas stream.

### Example 23

The procedure of Example 22 was repeated except that catalyst 18 was used in place of the catalyst. The result is shown in Table 7.

### Example 24

The procedure of Example 22 was repeated except that catalyst 19 was used in place of the catalyst. The result is shown in Table 7.

These results indicate that the dehydration temperature hardly affects the catalytic activity.

### Example 25

The procedure of Example 22 was repeated except that catalyst 20 was used in place of the catalyst. The result is shown in Table 7.

### Example 26

The procedure of Example 22 was repeated except that catalyst 21 was used in place of the catalyst. The result is shown in Table 7.

### Example 27

The procedure of Example 22 was repeated except that catalyst 22 wan used in place of the catalyst. The result is shown in Table 7. It indicates that the flow rate of the entering gas does not affect the catalytic activity by comparing the result of Example 27 to that of Example 22.

### Example 28

The procedure of Example 22 was repeated except that catalyst 23 was used in place of the catalyst. The result is shown in Table 7.

### Example 29

The procedure of Example 22 was repeated except that catalyst 24 was used in place of the catalyst. The result is shown in Table 7. The results in Example 22 - 29 indicate that dehydration by heating in a stream of argon, helium or air is effective.

**Table 7**

| | Catalyst | n' | Cy - Hex | Cy - HexOAc | |
|---|---|---|---|---|---|
| | | | Conversion (%) | Yield (%) | Selectivity (%) |
| Example 22 | 17 | 0 | 86.7 | 85.4 | 98.5 |
| Example 23 | 18 | 0 | 85.9 | 85.3 | 99.3 |
| Example 24 | 19 | 0 | 86.2 | 84.6 | 98.1 |
| Example 25 | 20 | 1.3 | 87.6 | 85.0 | 97.1 |
| Example 26 | 21 | 2.9 | 75.8 | 73.3 | 96.7 |
| Example 27 | 22 | 0 | 86.9 | 85.4 | 98.3 |
| Example 28 | 23 | 0 | 87.5 | 85.3 | 97.5 |
| Example 29 | 24 | 0 | 87.2 | 85.8 | 98.4 |

### Example 30

Preparation of a catalyst by heating and dehydrating dodecatungstosilicic acid in a gas stream:
In the muffle furnace as used in Example 21 was placed the above-mentioned dodecatungstosilicic acid. The heating and dehydrating treatment was effected at a prescribed temperature for a prescribed time while each gas was passed through the furnace at a prescribed flow rate.

The catalysts thus prepared, preparation conditions, and the number of molecules of water of crystallization (n') per one molecule or the heteropolyacid catalyst thus prepared are shown in Table 8.

**Table 8**

| | Gas | Flow rate (Nl/hr.) | Temperature (°C) | Time (hr.) | n' |
|---|---|---|---|---|---|
| Catalyst 25 | Ar | 5.0 | 370 | 3 | 0 |
| Catalyst 26 | Ar | 5.0 | 420 | 1 | 0 |
| Catalyst 27 | Ar | 5.0 | 300 | 3 | 0 |
| Catalyst 28 | Ar | 5.0 | 250 | 1 | 1.3 |
| Catalyst 29 | Ar | 5.0 | 250 | 0.5 | 2.9 |
| Catalyst 30 | Ar | 35.0 | 350 | 3 | 0 |
| Catalyst 31 | Air | 35.0 | 350 | 3 | 0 |
| Catalyst 32 | He | 35.0 | 350 | 3 | 0 |

### Example 31

The reaction of acetic acid with cyclohexene was effected under the same conditions as in Comparative Example 3 except that catalyst 25 (added 2.0 g) was used in place of the commercially available dodecatungstosilicic acid catalyst. As can be seen from the result in Table 9, the tungstosilicic acid catalyst thus heated and dehydrated in an argon gas stream has a high catalytic activity.

### Example 32

The procedure of Example 31 was repeated except that catalyst 26 was used in place of the catalyst. The result is shown in Table 9.

### Example 33

The procedure of Example 31 was repeated except that catalyst 27 was used in place of the catalyst. The result is shown in Table 9.

### Example 34

The procedure of Example 31 was repeated except that catalyst 28 was used in place of the catalyst. The result is shown in Table 9.

### Example 35

The prodedure of Example 31 was repeated except that catalyst 29 was used in place of the catalyst. The result is shown in Table 9.

### Example 36

The procedure of Example 31 was repeated except that catalyst 30 was used in place of the catalyst. The result is shown in Table 9. It indicates that when the gas flow rate is changed in preparing the catalyst (see Table 8), the catalytic activity is not affected comparing the result of Example 36 to that of Example 31.

### Example 37

The procedure of Example 31 was repeated except that catalyst 31 was used in place of the catalyst. The result is shown in Table 9.

### Example 38

The procedure of Example 31 was repeated except that catalyst 32 was used in place of the catalyst. The result is shown in Table 9.

The results obtained in Examples 31 - 38 show that argon, helium or air is effective as a gas which is passed through the preparation system upon preparing the catalyst by heating and dehydrating.

**Table 9**

| | Catalyst | n' | Cy - Hex | Cy - HexOAc | |
|---|---|---|---|---|---|
| | | | Conversion (%) | Yield (%) | Selectivity (%) |
| Example 31 | 25 | 0 | 92.6 | 90.5 | 97.8 |
| Example 32 | 26 | 0 | 91.9 | 90.2 | 98.1 |
| Example 33 | 27 | 0 | 90.6 | 90.0 | 99.4 |
| Example 34 | 28 | 1.3 | 92.2 | 90.7 | 98.4 |
| Example 35 | 29 | 2.9 | 77.3 | 74.4 | 96.2 |
| Example 36 | 30 | 0 | 91.8 | 90.2 | 97.7 |
| Example 37 | 31 | 0 | 93.1 | 90.8 | 97.5 |
| Example 38 | 32 | 0 | 91.5 | 90.3 | 98.7 |

### Example 39

The reaction was effected by repeating the procedure of Example 22 except that, after feeding the catalyst, acetic acid and cyclohexene, pure water was added so that the water amount in the reaction system was 1.1 mole per 1 mole of catalyst 17 (water of crystallization being zero). The result is shown is Table 10.
In Table 10, the result of Example 22 is also quoted for comparison.

### Example 40

The reaction was effected by repeating the procedure of Example 17 except that pure water was added in a way similar to Example 39 so that the water amount in the reaction system was 4.5 moles per 1 mole of catalyst 17 (water of crystallization being zero). The result is shown in Table 10.

### Example 41

The reaction was effected by repeating the procedure of Example 17 except that pure water was added in a way similar to Example 39 so that the water amount in the reaction system was 10.2 moles per 1 mole of catalyst 17 (water of crystallization being zero). The result is shown in Table 10.

### Example 42

The reaction was effected by repeating the procedure of Example 17 except that pure water was added in a way similar to Example 39 so that the water amount in the reaction system was 29.2 moles per 1 mole of catalyst 17 (water of crystallization being zero). The result is shown in Table 10.

### Example 43

The reaction was effected by repeating the procedure of Example 22 except that pure water was added in a way similar to Example 39 so that the water amount in the reaction system was 29.2 moles per 1 mole of catalyst 17 (water of crystallization being zero). The result is shown in Table 10. The result of Example 43 shows that an improvement in catalytic activity due to dehydrating the catalyst was not observed.

### Example 44

The reaction was carried out by repeating the procedure of Example 25 except that, after feeding the catalyst, acetic acid and cyclohexene, pure water was added so that the amount of water other than water of crystallization of catalyst 20 (n' = 1.3) was 10.2 moles per one mole of the catalyst in the reaction system. The result is shown in Table 10.

### Example 45

The reaction was carried out under the same conditions as in Example 44 except that catalyst 21 (n' = 2.9) was used in place of the catalyst. The result is shown in Table 10.

### Example 46

The reaction was effected under the same conditions as in Example 31 except that pure water was added in a way similar to Example 39 so that the water amount per one molecule of catalyst 25 (water of crystallization being zero) was 1.1 molecule. The result is shown in Table 10. The result of Example 31 is also listed in Table 10.

### Example 47

The reaction was carried out under the same conditions as in Example 57 except that the amount of added pure water was 4.5 molecules per one molecule of catalyst 25 (water of crystallization being zero) in place of that in Example 57. The result is shown in Table 10.

### Example 48

The reaction was carried out under the same conditions as in Example 46 except that the amount of added pure water was 10.5 molecules per one molecule of the catalyst in place of that in Example 46. The result is shown in Table 10.

### Example 49

The reaction was carried out under the same conditions as in Example 46 except that the amount of added pure water was 29.2 molecules per one molecule of the catalyst in place of that in Example 46. The result is shown in Table 10.

### Comparative Example 11

The reaction was carried out under the same conditions as in Example 46 except that the amount of added pure water was 35.4 molecules per one molecule of the catalyst in place of that in Example 46. The result is shown in Table 10. This indicates that there was no improvement in the catalytic activity due to the preparation of the catalyst by heating and dehydration.

### Example 50

The procedure of Example 34 was repeated except that the amount of water other than water of crystallization contained in catalyst 28 (n' = 1.3) was 10.2 moles per one mole of the catalyst. The result is shown in Table 10.

### Example 51

The procedure of Example 50 was repeated except that catalyst 29 was used in place of the catalyst. The result is shown in Table 10.

**Table 10**

| | Catalyst | Water (a) | Cy - Hex | Cy - HexOAc | |
|---|---|---|---|---|---|
| | | | Conversion (%) | Yield (%) | Selectivity (%) |
| Example 22 | 17 | 0.02 | 86.7 | 85.4 | 98.5 |
| Example 39 | 17 | 1.1 | 86.9 | 85.7 | 98.6 |
| Example 40 | 17 | 4.5 | 82.1 | 80.6 | 98.2 |
| Example 41 | 17 | 10.2 | 78.3 | 77.6 | 99.1 |
| Example 42 | 17 | 29.2 | 46.9 | 45.3 | 96.6 |
| Example 43 | 17 | 34.3 | 33.7 | 32.9 | 97.6 |
| Example 44 | 20 | 10.2 | 78.5 | 78.0 | 99.4 |
| Example 45 | 21 | 10.2 | 77.2 | 76.1 | 98.6 |
| Example 31 | 25 | 0.02 | 92.6 | 90.5 | 97.8 |
| Example 46 | 25 | 1.1 | 91.8 | 90.3 | 98.4 |
| Example 47 | 25 | 4.5 | 88.9 | 85.7 | 96.4 |
| Example 48 | 25 | 10.5 | 80.3 | 78.8 | 98.1 |
| Example 49 | 25 | 29.0 | 67.2 | 65.5 | 97.5 |
| Comparative Example 11 | 25 | 35.4 | 48.4 | 46.2 | 95.5 |
| Example 50 | 28 | 10.2 | 80.5 | 79.4 | 98.6 |
| Example 51 | 29 | 10.2 | 71.2 | 69.1 | 97.1 |
| (a) : The number of molecules of water other than water of crystallization of the catalyst present in the reaction system per one molecule of the catalyst. | | | | | |

## Claims

1. A process for producing cyclohexyl acetate which comprises reacting acetic acid with cyclohexene in the presence of at least one heteropolyacid mainly composed of oxides of tungsten which may have water of crystallization adjusted to an amount on average of 3.0 molecules or less per one molecule of the heteropolyacid.

2. The process according to claim 1 in which the heteropolyacid is a heteropolyacid mainly composed of oxides of tungsten containing no water of crystallization.

3. The process according to claim 1 or 2 in which the heteropolyacid is mainly composed of oxides of tungsten of the general formula,
(M₁)ₐ(M₂)_{b}(W)_{c}(O)_{d}(H)ₑ
where M₁ and M₂ are independently metal elements, W is tungsten, O is oxygen, H is hydrogen, a is an integer of 1 or 2, b is an integer of 0, 1 or 2, c is a positive integer of 20 or less, d is a positive integer of 100 or less, and e is a positive integer of 10 or less.

4. The process according to claim 3 in which M₁ is an element selected from the group of P, Si, Co, Mn, Ni, As, Fe, V, and B, and M₂ is V.

5. The process according to claim 1 in which the heteropolyacid is prepared by heating a heteropolyacid mainly composed of oxides of tungsten at a temperature range of 150°C to 500 °C to adjust the amount of water of crystallization contained therein to an average of 3.0 molecules or less per one molecule of the heteropolyacid.

6. The process according to claim 1 or 2 in which the heteropolyacid is prepared by heating a heteropolyacid mainly composed of oxides of tungsten in a stream of a gas inert to the heteropolyacid to adjust the amount of water of crystallization contained therein to an average of 3.0 molecules or less per one molecule of the heteropolyacid.

7. The process according to claim 1 or 2 in which the reaction is carried out under the condition that the amount of water other than water of crystallization of the heteropolyacid mainly composed of tungstenoxide is 30.0 molecules or less per one molecule of the heteropolyacid.

8. The process according to any claims 1-4 in which the heteropolyacid mainly composed of oxides of tungsten is at least one of tungstophosphoric acid and tungstosilicic acid.

## Patentansprüche

1. Verfahren zur Herstellung von Essigsäurecyclohexylester, umfassend die Umsetzung von Essigsäure mit Cyclohexen in Gegenwart mindestens einer hauptsächlich aus Wolframoxiden bestehenden Heteropolysäure, die Kristallwasser, eingestellt auf eine durchschnittliche Menge von 3,0 Moleküle oder Weniger pro Molekül der Heteropolysäure, aufweisen können.

2. Verfahren nach Anspruch 1, wobei die Heteropolysäure eine hauptsächlich aus Wolframoxiden bestehende Heteropolysäure ist, die kein Kristallwasser enthält.

3. Verfahren nach Anspruch 1 oder 2, wobei die Heteropolysäure hauptsächlich aus Wolframoxiden der allgemeinen Formel
(M₁)ₐ(M₂)_{b}(W)_{c}(O)_{d}(H)ₑ
besteht, in der M₁ und M₂ unabhängig Metallelemente sind, W Wolfram ist, O Sauerstoff ist, H Wasserstoff ist, a eine ganze Zahl von 1 oder 2 ist, b eine ganze Zahl von 0, 1 oder 2 ist, c eine positive ganze Zahl von 20 oder weniger ist, d eine positive ganze Zahl von 100 oder weniger ist und e eine positive ganze Zahl von 10 oder weniger ist.

4. Verfahren nach Anspruch 3, wobei M₁ ein Element, ausgewählt aus der Gruppe P, Si, Co, Mn, Ni, As, Fe, V und B, und M₂ V ist.

5. Verfahren nach Anspruch 1, wobei die Heteropolysäure durch Erhitzen einer hauptsächlich aus Wolframoxiden bestehenden Heteropolysäure auf einen Temperaturbereich von 150°C bis 500°C zur Einstellung der Menge des darin enthaltenen Kristallwassers auf ein Mittel von 3.0 Moleküle oder weniger pro Molekül Heteropolysäure, hergestellt wird.

6. Verfahren nach Anspruch 1 oder 2, wobei die Heteropolysäure durch Erhitzen einer hauptsächlich aus Wolframoxiden bestehenden Heteropolysäure in einem Strom eines zur Heteropolysäure inerten Gases hergestellt wird, um die Menge des darin enthaltenen Kristallwassers auf ein Mittel von 3.0 Moleküle oder weniger pro Molekül der Heteropolysäure einzustellen.

7. Verfahren nach Anspruch 1 oder 2, wobei die Umsetzung unter der Bedingung durchgeführt wird, daß die Menge an anderem Wasser als Kristallwasser der hauptsächlich aus Wolframoxid bestehenden Heteropolysäure 30.0 Moleküle oder weniger pro Molekül der Heteropolysäure beträgt.

8. Verfahren nach einem der Ansprüche 1 - 4, wobei die hauptsächlich aus Wolframoxiden bestehende Heteropolysäure mindestens eine Wolframatophosphorsäure und Wolframatokieselsäure ist.

## Revendications

1. Procédé pour produire l'acétate de cyclohexyle, ce procédé comprenant la réaction de l'acide acétique avec le cyclohexène en présence d'au moins un hétéropolyacide principalement composé d'oxydes du tungstène qui peut comporter de l'eau de cristallisation dont la teneur a été ajustée à une quantité égale ou inférieure en moyenne à 3,0 molécules pour 1 molécule de l'hétéropolyacide.

2. Procédé selon la revendication 1, dans lequel l'hétéropolyacide est un hétéropolyacide principalement composé d'oxydes du tungstène ne contenant pas d'eau de cristallisation.

3. Procédé selon la revendication 1 ou 2, dans lequel l'hétéropolyacide est principalement composé d'oxydes du tungtène de formule générale,
(M₁)ₐ(M₂)_{b}W)_{c}(O)_{d}(H)ₑ
dans laquelle M₁ et M₂ sont, indépendamment, des éléments métalliques, W représente le tungstène, O représente l'oxygène, H représente l'hydrogène, a est un nombre entier valant 1 ou 2, b est un nombre entier valant 0, 1 ou 2, c est un nombre entier positif égal ou inférieur à 20, d est un nombre entier positif égal ou inférieur à 100, et e est un nombre entier positif égal ou inférieur à 10.

4. Procédé selon la revendication 3, dans lequel M₁ est un élément choisi dans l'ensemble formé par P, Si, Co, Mn, Ni, As, Fe, V et B, et M₂ est V.

5. Procédé selon la revendication 1, dans lequel on prépare l'hétéropolyacide en soumettant un hétéropolyacide, principalement composé d'oxydes de tungstène, à du chauffage à une température comprise entre 150°C et 500°C pour ajuster la quantité d'eau de cristallisation contenue dans ce composé et la porter à une valeur moyenne égale ou inférieure à 3,0 molécules pour 1 molécule de l'hétéropolyacide.

6. Procédé selon la revendication 1 ou 2, dans lequel on prépare l'hétéropolyacide en soumettant un hétéropolyacide, principalement composé d'oxydes du tungstène, à du chauffage dans un courant de gaz inerte à l'égard de l'hétéropolyacide pour ajuster la quantité d'eau de cristallisation contenue dans cet hétéropolyacide à une valeur moyenne égale ou inférieure à 3,0 molécules pour 1 molécule de l'hétéropolyacide.

7. Procédé selon la revendication 1 ou 2, dans lequel on effectue la réaction de façon que la quantité d'eau, autre que l'eau de cristallisation de l'hétéropolyacide principalement composé d'oxydes du tungstène, soit égale ou inférieure à 30,0 molécules (d'eau) pour 1 molécule de l'hétéropolyacide.

8. Procédé selon l'une quelconque des revendications 1 à 4, dans lequel l'hétéropolyacide principalement composé d'oxydes du tungstène est au moins un acide choisi parmi de l'acide tungstophosphorique et de l'acide tungstosilicique.
